# EUROPEAN PATENT APPLICATION

(11) **EP 4 741 793 A1**
(43) Date of publication of application: **13.05.2026**
(21) Application number: 24835943.2
(22) Date of filing: 24.06.2024
(51) Int. Cl.: G01M 99/00, F24F 11/49

(54) **PERFORMANCE MEASUREMENT METHOD FOR AIR CONDITIONER, PERFORMANCE MEASUREMENT DEVICE FOR AIR CONDITIONER, AND CONTROL METHOD FOR CONDITION GENERATOR**

(30) Priority: 05.07.2023 JP 2023110460
(71) Applicant: WASEDA UNIVERSITY, Shinjuku-ku Tokyo 169-8050 (JP)
(72) Inventor: SAITO, Kiyoshi, Tokyo 169-8050 (JP); GIANNETTI, Niccolo, Tokyo 169-8050 (JP)
(74) Representative: Regimbeau
(86) International application number: PCT/JP2024/022837
(87) International publication number: WO 2025/009430

(57) **Abstract**

The invention provides a method for measuring the performance of an air conditioner that achieves an improvement in the accuracy of performance measurement of a dynamically controlled air conditioner. A method for measuring the performance of an air conditioner 2 comprises: emulating the air state of a virtual room, the virtual room being a virtual reproduction of an actual room, using the air state of the air blown out from the air conditioner 2 to determine future changes in the air state of the virtual room; compensating for a delay in the air state of the test room relative to a setting of the condition generator 4 in the case of using the future change in the air state of the virtual room for the setting of the condition generator; controlling the condition generator 4 using, as the setting, the future change in the air state of the virtual room for which the delay has been compensated; and measuring the performance of the air conditioner in the test room under the measurement conditions generated by the condition generator 4.

## Description

### TECHNICAL FIELD

The present invention relates to a method and apparatus for measuring the performance of an air conditioner, and a method for controlling a condition generator.

### BACKGROUND ART

As a simple method for measuring the performance of an air conditioner, a method is known in which the performance is measured by driving the air conditioner in a stable condition without performing capacity control by fixing the rotation speed of the compressor. However, since such performance measurement in a fixed operating state, that is, a static operating state is not carried out under actual operating conditions, there is a risk that a discrepancy may arise between the measurement results and the performance in the actual use environment.

Therefore, performance measurement while dynamically changing the operating state of the air conditioner has been studied. Such performance measurement under a dynamic operating state is also sometimes referred to as load-based performance measurement. According to the technology disclosed in Patent Literature 1, a predetermined test environment, such as a test room, is prepared to measure the performance of an air conditioner in a dynamic operating state. This technology involves measuring the performance of an air conditioner in a dynamic operating state after controlling electrical equipment, human body models, and the like placed in the test room in consideration of the actual use environment, such as heat generation from the electrical equipment, living heat inside the room and the like.

### LIST OF CITATIONS

### Patent Literature

[Patent Literature 1] JP 2007-333557 A

### SUMMARY OF INVENTION

### PROBLEM TO BE SOLVED BY THE INVENTION

The technology disclosed in Patent Literature 1 requires the construction of identical test rooms at each location to ensure identical implementation environments when the performance measurement is implemented at different sites. However, it is difficult to reproduce a completely identical test environment, including the external environment, which may lead to variations in the performance measurement results.

Therefore, a method has been studied in which a condition generator configured by an air conditioner different from the air conditioner to be measured, is installed in the test room. This method emulates a virtual test room (virtual room) based on the state of the air blown out from the air conditioner to be measured, followed by the performance measurement of the air conditioner to be measured while controlling the condition generator so that the actual air state of the test room matches the air state of the virtual room derived from the emulation. This method can exclude factors that depend on the environment such as the heat capacity of the test room and thus can measure the performance of the air conditioner in a dynamic operating state while achieving a highly reproducible measurement environment.

However, even with this method involving emulation, delays may occur due to the control of the condition generator and elements such as the thermal capacity of the air in the test room, which may cause the change (response) of the actual test room state to lag behind the virtual room state derived from the emulation. As a result, there has been a problem that these delays can affect the results of air conditioner performance measurement under dynamic operating conditions.

The present invention has been made in view of the above-described problem, and an object thereof is to provide a method and apparatus for measuring the performance of an air conditioner and a method for controlling a condition generator, which achieves an improvement in the accuracy of performance measurement of the air conditioner in a dynamic operating state.

### MEANS TO SOLVE THE PROBLEM

The method for measuring the performance of an air conditioner according to the present invention measures the performance of an air conditioner dynamically operated in a test room equipped with a condition generator that generates measurement conditions. This method for measuring the performance of an air conditioner comprises: emulating the air state of a virtual room, the virtual room being a virtual reproduction of an actual room, using the air state of the air blown out from the air conditioner to determine future changes in the air state of the virtual room; compensating for a delay in the air state of the test room relative to a setting of the condition generator in the case of using the future change in the air state of the virtual room for the setting of the condition generator; controlling the condition generator using, as the setting, the future change in the air state of the virtual room for which the delay has been compensated; and measuring the performance of the air conditioner in the test room under the measurement conditions generated by the condition generator.

The apparatus for measuring the performance of an air conditioner according to the present invention measures the performance of an air conditioner dynamically operated in a test room equipped with a condition generator that generates measurement conditions. The apparatus for measuring the performance of an air conditioner comprises: an emulation unit configured to emulate the air state of a virtual room, the virtual room being a virtual reproduction of an actual room, using the air state of the air blown out from the air conditioner to determine future changes in the air state of the virtual room; a delay compensation unit configured to compensate for a delay in the air state of the test room relative to a setting of the condition generator in the case of using the future change in the air state of the virtual room for the setting of the condition generator and to control the condition generator using, as the setting, the future change in the air state of the virtual room for which the delay has been compensated; and an air conditioning performance measurement unit configured to measure the performance of the air conditioner in the test room under the measurement conditions generated by the condition generator.

The method for controlling a condition generator according to the present invention controls a condition generator that is installed in a test room and generates conditions for performance measurement of an air conditioner dynamically operated. This method for controlling a condition generator comprises: emulating the air state of a virtual room, the virtual room being a virtual reproduction of an actual room, using the air state of the air blown out from the air conditioner to determine future changes in the air state of the virtual room; compensating for a delay in the air state of the test room relative to a setting of the condition generator in the case of using the future change in the air state of the virtual room for the setting of the condition generator; and controlling the condition generator using, as the setting, the future change in the air state of the virtual room for which the delay has been compensated.

### EFFECT OF THE INVENTION

The method and apparatus for measuring the performance of an air conditioner and the method for controlling a condition generator of the present invention compensate for a delay in the change of the air state of the test room relative to the set value input to the condition generator with respect to the air state of the virtual room derived from emulation. This delay compensation reduces the difference between the air state of the test room and that of the virtual room when measuring the performance of a dynamically controlled air conditioner thereby improving the accuracy of the performance measurement.

### BRIEF EXPLANATION OF FIGURES

[FIG. 1] FIG. 1 is a schematic configuration diagram of a test room for measuring the performance of an air conditioner, common to each embodiment.
[FIG. 2] FIG. 2 is an explanatory diagram of the operation of the virtual room calculation unit of the first embodiment.
[FIG. 3] FIG. 3 is an explanatory diagram of the operation of a virtual room calculation unit of a comparative example.
[FIG. 4] FIG. 4 is a graph showing the performance of the delay model generated in the first example.
[FIG. 5] FIG. 5 is a graph showing the performance of the generated compensation model.
[FIG. 6] FIG. 6 is a graph showing the performance of the compensation model generated in the second example.
[FIG. 7] FIG. 7 is a graph showing measurement results when actually operated in the test room using the generated delay model.
[FIG. 8] FIG. 8 is an explanatory diagram of the operation of the virtual room calculation unit of the second embodiment.

### EMBODIMENTS

Embodiments of the present invention will be described in detail below with reference to the drawings. In the following description, the same constituent elements are denoted by the same reference numerals, and redundant explanations are omitted.

### (First Embodiment)

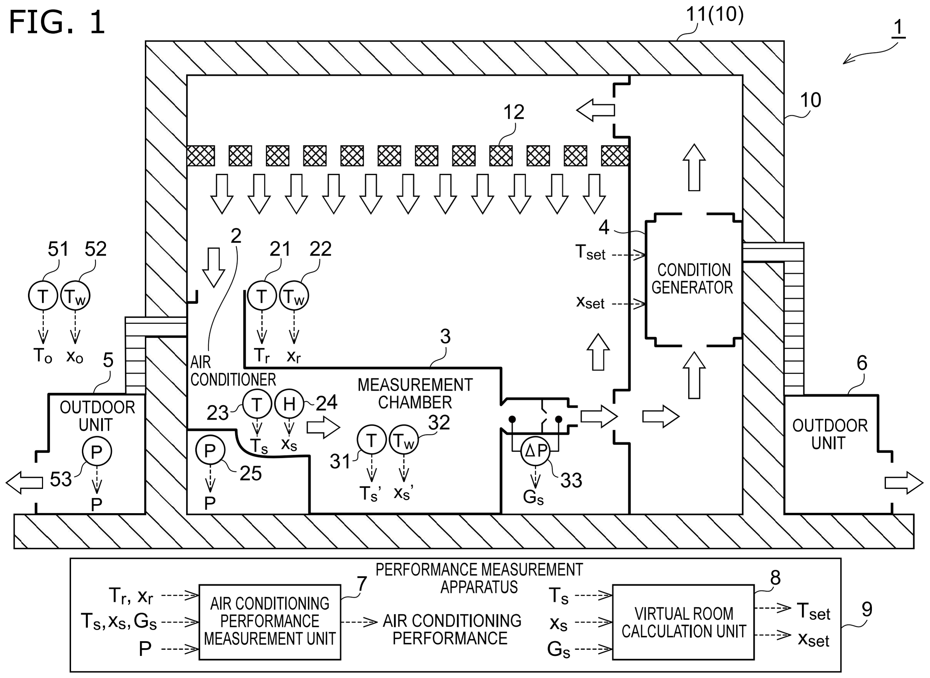

FIG. 1 is a schematic configuration diagram of a test room 1. The illustrated test room 1 has a configuration common to each embodiment. As illustrated, the test room 1 is provided with: an air conditioner 2 to be measured, a measuring chamber 3 that captures the air blown out from the air conditioner 2, and a condition generator 4 that takes in the air blown out from the measuring chamber 3 and generates the conditions for performance measurement by setting the air state (temperature and humidity) inside the test room 1 to a predetermined air state. As an example, the condition generator 4 is an air conditioner different from the air conditioner 2 and is capable of controlling temperature and humidity. The air conditioner 2 operates integrally with an outdoor unit 5, and the condition generator 4 operates integrally with an outdoor unit 6.

The air blown out from the air conditioner 2 blows into the measuring chamber 3. Furthermore, part of the air blown out from the measuring chamber 3 blows into the condition generator 4 while the rest blows into the test room 1. Alternatively, all of the air blown out from the measuring chamber 3 may blow into the condition generator 4. The temperature and humidity of the air blown into the condition generator 4 are changed by the condition generator 4.

Two control units, an air conditioning performance measurement unit 7 for executing performance measurement for the air conditioner 2, and a virtual room calculation unit 8 for determining a target value for the condition generator 4 are provided integrally in a performance measurement apparatus 9. The performance measurement apparatus 9 is composed of a calculator (computer) equipped with a central processing unit (CPU), a memory unit, and the like. The performance measurement apparatus 9 executes processing to realize the functions of the air conditioning performance measurement unit 7 and the virtual room calculation unit 8 by executing a specific program stored in the memory unit. Note that the air conditioning performance measurement unit 7 and the virtual room calculation unit 8 may be configured by a single calculator or by a plurality of calculators.

The air conditioning performance measurement unit 7 is configured to calculate the air conditioning performance of the air conditioner 2 from the measured air state inside the test room 1 and the power consumption of the air conditioner 2. Since the air conditioner 2 is dynamically controlled according to the measurement conditions, the air conditioning performance measurement unit 7 can execute dynamic performance measurement of the air conditioner 2.

The virtual room calculation unit 8 predicts the air state (temperature, humidity) of a room virtually created (hereinafter referred to as "virtual room") by emulation using the measured air state of the air blown out from the air conditioner 2 and controls the condition generator 4 so that the air state of the test room 1 matches the air state of the virtual room. The use of the condition generator 4 to make the air state of the test room 1 match the air state of the virtual room as described can suppress variations in the results of air conditioning performance measurement caused by differences in the configuration of a plurality of test rooms 1 installed in different locations.

The test room 1 is entirely covered by a heat insulation material 10. The test room 1 has a double-layer structure in which an inner ceiling 12 is provided inside an outer wall 11 of the ceiling and is configured to allow the air blown out from the condition generator 4 to be taken in between the outer wall 11 and the inner ceiling 12. The inner ceiling 12 is provided with a plurality of holes, and the air blown out from the condition generator 4 is diffused throughout the inside of the test room 1 via these holes. Note that, although the inner ceiling 12 is provided inside the test room 1 in the present embodiment, the inner ceiling 12 may not be provided, and the air blown out from the condition generator 4 may be blown directly or indirectly into the test room 1.

The air conditioner 2 is connected via piping or the like to the outdoor unit 5 installed outside the test room 1. The outdoor unit 5 is equipped with a compressor or the like, and the air conditioner 2 and the outdoor unit 5 operate as an integral unit so that air at a predetermined temperature, humidity, and airflow rate is allowed to be blown out from the air conditioner 2.

A thermometer 21 for measuring the room temperature Tᵣ inside the test room 1, and a wet-bulb thermometer 22 for measuring humidity are installed in the vicinity of the air intake port of the air conditioner 2. The room humidity xᵣ is determined using the wet-bulb temperature measured by the wet-bulb thermometer 22 and the room temperature Tᵣ measured by the thermometer 21. A thermometer 23 for measuring the blowing temperature Tₛ of the air blown out from the air conditioner 2, and a hygrometer 24 for directly measuring humidity are also installed in the vicinity of the air outlet port of the air conditioner 2. Since a temperature and humidity distribution exists at the air outlet port of the air conditioner 2, it is preferable that the hygrometer 24 is installed to directly measure the humidity xₛ of the blowing air, in place of a wet-bulb thermometer that measures humidity by comparing with the temperature measured by a thermometer.

Furthermore, the air conditioner 2 is controlled integrally with the outdoor unit 5, and the power consumption P used for air conditioning of the test room 1 is determined using a power meter 25 provided in the air conditioner 2 and a power meter 53 provided in the outdoor unit 5. A thermometer 51 and a wet-bulb thermometer 52 are provided in the vicinity of the outdoor unit 5, which respectively measure the outdoor temperature Tₒ and the outdoor humidity xₒ outside the test room 1. The outdoor temperature Tₒ and the outdoor humidity xₒ are not used in the first embodiment but are used in the second embodiment. Note that a configuration could be used in which a power meter is provided on either the air conditioner 2 or the outdoor unit 5.

A thermometer 31 and a wet-bulb thermometer 32 are provided inside the measuring chamber 3. An air flow meter 33 is provided at the outlet of the measuring chamber 3. The air flow meter 33 determines the differential pressure between two pressure gauges provided along the air flow and calculates the air volume Gₛ blown out from the air conditioner 2 based on the differential pressure.

Here, since a temperature and humidity distribution exists in the vicinity of the air outlet port of the air conditioner 2, errors may occur in the temperature Tₛ and humidity xₛ acquired using the thermometer 23 and the hygrometer 24. In contrast, the temperature Tₛ^{'} and humidity xₛ' determined by the thermometer 31 and the wet-bulb thermometer 32 may have errors relative to the actual state of the air blown out from the air conditioner 2, when compared to the blowing temperature Tₛ and blowing humidity xₛ measured by the thermometer 23 and the hygrometer 24 provided near the air outlet of the air conditioner 2. This is because the measuring chamber 3 is located at a distance from the air outlet of the air conditioner 2, and the measuring chamber 3 itself has heat capacity. However, since the blowing air is agitated within the measuring chamber 3, a more stable blowing air state measurement can be achieved.

Therefore, the measurement accuracy of the blowing temperature Tₛ and the blowing humidity xₛ can be enhanced by calibrating the blowing temperature Tₛ and the blowing humidity xₛ measured by the thermometer 23 and the hygrometer 24, using the temperature Tₛ' and the humidity xₛ^{'} determined by the thermometer 31 and the wet-bulb thermometer 32 in a steady state in advance. Furthermore, in cases where the measurement delay resulting from the distance between the air outlet port of the air conditioner 2 and the measuring chamber 3 can be regarded as relatively small, the blowing temperature Tₛ and blowing humidity xₛ from the air conditioner 2 may be determined by measuring the air state of the measuring chamber 3 using the thermometer 31 and the wet-bulb thermometer 32, without using the thermometer 23 and the hygrometer 24.

The air conditioning performance measurement unit 7 receives inputs of: the room temperature Tᵣ and the room humidity xᵣ acquired using the thermometer 21 and the wet-bulb thermometer 22, respectively; the blowing temperature Tₛ, the blowing humidity xₛ (which may be calibrated by the temperature Tₛ^{'} and the humidity xₛ' acquired by the thermometer 31 and the wet-bulb thermometer 32), and the blowing air volume Gₛ, acquired using the thermometer 23, the hygrometer 24, and the air flow meter 33, respectively; and the power consumption P measured by the power meters 25, 53.

The air conditioning performance measurement unit 7 calculates the enthalpy of the room air from the room temperature Tᵣ and the room humidity xᵣ, calculates the enthalpy of the air blown out from the air conditioner 2 using the blowing temperature Tₛ and the blowing humidity xₛ, and calculates the difference between the two enthalpies. Then, the air conditioning performance measurement unit 7 determines the air conditioning capacity (cooling capacity/heating capacity) by multiplying the enthalpy difference by the blowing air volume Gₛ. Finally, the air conditioning performance measurement unit 7 calculates the air conditioning performances of the air conditioner 2 and the outdoor unit 5 by dividing the determined air conditioning capacity by the power consumption P.

The virtual room calculation unit 8 receives inputs of the blowing temperature Tₛ, the blowing humidity xₛ, and the blowing air volume Gₛ acquired using the thermometer 23, the hygrometer 24, and the air flow meter 33, and uses these inputs to emulate the air state of the virtual room. Then, the virtual room calculation unit 8 transmits set values (set temperature Tₛₑₜ, set humidity xₛₑₜ) to the condition generator 4, such that the air state of the test room 1 matches the air state of the virtual room derived from the emulation. This enables measurement of the air conditioning performance of the air conditioner 2 in the test room 1, where the air condition matches that of the virtual room.

The condition generator 4 is configured by an air conditioner different from the air conditioner 2 to be measured and operates integrally with the outdoor unit 6 connected via piping or the like. The condition generator 4 takes in part or all of the air blown out from the measuring chamber 3 and operates according to the set temperature Tₛₑₜ and set humidity xₛₑₜ from the virtual room calculation unit 8.

Additionally, thermometers, hygrometers, and wet-bulb thermometers other than the thermometers 21, 23, and 51, the hygrometer 24, and the wet-bulb thermometers 22 and 52 may be provided inside and outside the test room 1. The use of the measurementresults from a plurality of thermometers, hygrometers, and wet-bulb thermometers can determine the room temperature Tᵣ and room humidity xᵣ, the blowing temperature Tₛ and blowing humidity xₛ, and the outdoor temperature Tₒ and outdoor humidity xₒ with higher accuracy.

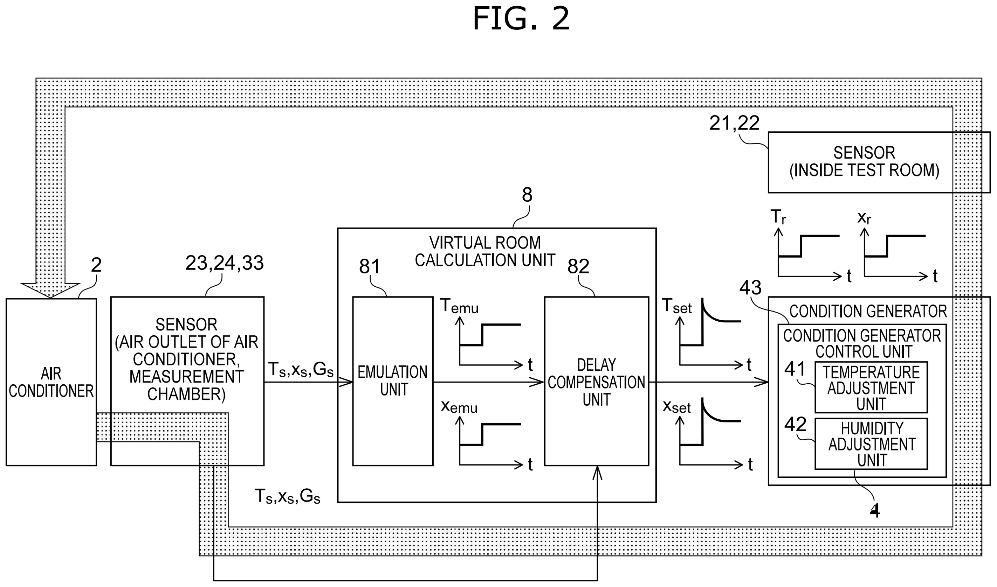

FIG. 2 is an explanatory diagram of the operation of a virtual room calculation unit 8 of the present embodiment. This figure indicates the flow of control by thin arrows, and the flow of air by thick arrows (with dotted hatching).

As indicated by the thick arrows, the air blown out from the air conditioner 2 passes through the measuring chamber 3, and after being modified to predetermined conditions (temperature, humidity) by the condition generator 4, is returned into the test room 1 again, and is sucked into the air conditioner 2.

Meanwhile, as indicated by the thin arrows, the blowing temperature Tₛ, blowing humidity xₛ, and blowing airflow rate Gₛ measured by sensors (thermometer 23, hygrometer 24, and air flow meter 33) provided at the outlet port of the air conditioner 2 and in the measuring chamber 3 are input to the virtual room calculation unit 8. The virtual room calculation unit 8 comprises an emulation unit 81 and a delay compensation unit 82. Note that in the present embodiment, an example has been described in which the emulation unit 81 and the delay compensation unit 82 integrally constitute the virtual room calculation unit 8, but the emulation unit 81 and the delay compensation unit 82 may be independent and different computers or software.

The emulation unit 81 receives the measured blowing temperature Tₛ, blowing humidity xₛ, and blowing airflow rate Gₛ, and emulates the air state of the virtual room to determine a virtual room temperature Tₑₘᵤ and a virtual room humidity xₑₘᵤ. This emulation reproduces the amount of heat into and out of the virtual room, specifically incident solar radiation, the state of ventilation with the exterior (including drafts), and living heat generated by occupants and electrical appliances inside.

More specifically, the emulation unit 81 uses the various parameters of the state of the air blown out from the air conditioner 2, that is, the blowing temperature Tₛ, the blowing humidity xₛ, and the blowing air volume Gₛ, to solve differential equations in consideration of the heat transfer into and out of the virtual room and predict the temporal change in the air state (temperature, humidity) of the virtual room. These future predicted values of the air state in the virtual room are used to control the condition generator 4 after being corrected by the subsequent delay compensation unit 82. These future predicted values of the air state in the virtual room indicate the air state that changes over time and are denoted by the virtual room temperature Tₑₘᵤ and the virtual room humidity xₑₘᵤ.

The delay compensation unit 82 receives the blowing temperature Tₛ, the blowing humidity xₛ, and the blowing air volume Gₛ measured by the sensors, similar to the emulation unit 81, in addition to the virtual room temperature Tₑₘᵤ and the virtual room humidity xₑₘᵤ output from the emulation unit 81.

Whereupon, due to the mechanical control of the condition generator 4 and the thermal capacity, etc., of the air within the test room 1, the change (response) of the air state (room temperature Tᵣ, room humidity xᵣ) within the test room 1 is delayed relative to the settings (set temperature Tₛₑₜ, set humidity xₛₑₜ) input to the condition generator 4.

Therefore, the delay compensation unit 82 performs a correction so as to compensate for the delay with respect to the virtual room temperature Tₑₘᵤ and the virtual room humidity xₑₘᵤ output from the emulation unit 81, by feedforward control using the input blowing temperature Tₛ, blowing humidity xₛ, and blowing air volume Gₛ. The delay compensation unit 82 outputs the set temperature Tₛₑₜ and the set humidity xₛₑₜ, for which the delay has been compensated, to the condition generator 4, and the condition generator 4 is controlled accordingly.

Furthermore, since the control target of the condition generator 4 is the air state of the test room 1 and thus uncertainty exists in the control, a control error may arise in the air state. For this reason, the delay compensation unit 82 may also be configured to compensate for such errors.

The condition generator 4 has a temperature adjustment unit 41 and a humidity adjustment unit 42 and operates based on the set temperature Tₛₑₜ and the set humidity xₛₑₜ output from the delay compensation unit 82. The condition generator 4 operating in this way can allow the room temperature Tᵣ and the room humidity xᵣ of the test room 1 to be brought closer to the virtual room temperature Tₑₘᵤ and the virtual room humidity xₑₘᵤ. Note that the temperature adjustment unit 41 and the humidity adjustment unit 42 are control units of the condition generator 4 and may be configured integrally as a condition generator control unit 43.

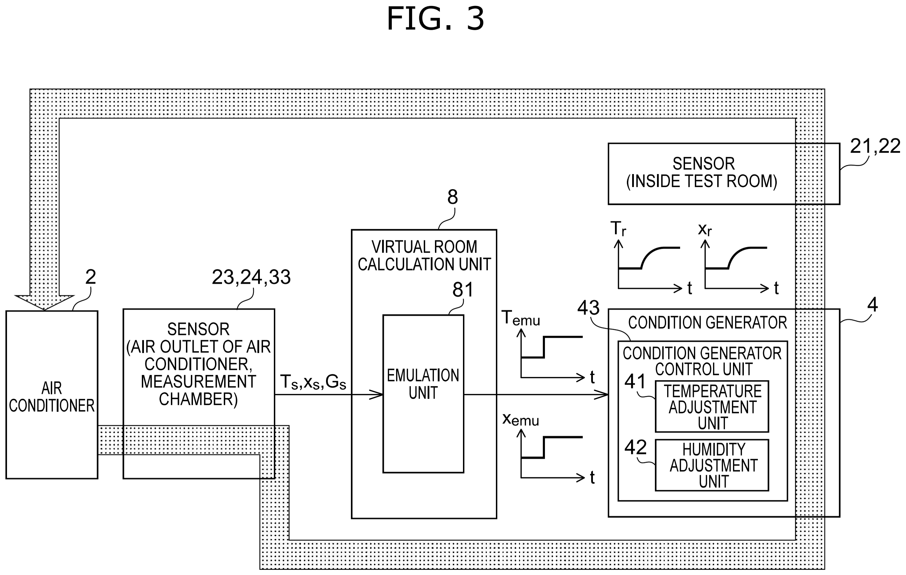

FIG. 3 is an explanatory diagram of the operation of a virtual room calculation unit 8 of a comparative example. The configuration illustrated in this figure is different from the virtual room calculation unit 8 of the first embodiment illustrated in FIG. 2 in that the delay compensation unit 82 within the virtual room calculation unit 8 is omitted.

Since the delay compensation unit 82 is omitted, the condition generator 4 is controlled using the state of the virtual room (virtual room temperature Tₑₘᵤ, virtual room humidity xₑₘᵤ) determined by the emulation unit 81 as the set value. Such control causes delays occurring due to the mechanical control of the condition generator 4, the heat capacity of the air inside the test room 1, and the like, and therefore the state (room temperature Tᵣ, room humidity xᵣ) inside the test room 1 is delayed relative to the set values.

Therefore, in the present embodiment, a delay compensation unit 82 provided downstream of the emulation unit 81 as illustrated in FIG. 2 enables the set temperature Tₛₑₜ and set humidity xₛₑₜ, for which delays have been compensated, to be used for controlling the condition generator 4. As a result, the air state of the test room 1 (room temperature Tᵣ, room humidity xᵣ) can be brought close to the air state of the virtual room (virtual room temperature Tₑₘᵤ, virtual room humidity xₑₘᵤ) determined by the emulation unit 81.

The delay compensation unit 82 may be configured, for example, by the following method. First of all, after generating a delay model that indicates the delay of the air state of the test room 1 relative to the set values of the condition generator 4, a compensation model that compensates for that delay is generated. Then, the delay compensation unit 82 is configured using the compensation model thus generated.

In detail, first of all, a delay model is generated which models the delay that occurs in a case where the delay compensation unit 82 does not exist, such as in the comparative example of FIG. 3. For example, the delay model is configured by machine learning using: past data of the settings (set temperature Tₛₑₜ, set humidity xₛₑₜ) of the condition generator 4; past data of the air state (virtual room temperature Tₑₘᵤ, virtual room humidity xₑₘᵤ) of the virtual room; past data of the air state (room temperature Tᵣ, room humidity xᵣ) of the test room 1; and past data of the air state (blowing temperature Tₛ, blowing humidity xₛ, blowing air volume Gₛ) of the air blown out from the air conditioner 2, and the like.

Next, a compensation model that compensates for the delay is generated using the generated delay model. For example, when one of the inputs to the delay model is changed, the output value changes according to the change in the input. One of the changing output values is the air state of the test room 1. Therefore, a change in the input is determined such that the deviation of the air state of the test room 1 after the change with respect to the air state of the virtual room is minimized over time. For example, the use of a genetic algorithm allows such input changes to be repeated to determine the optimal change. Finally, a compensation model that compensates for the determined change can be generated.

In this way, the delay model is generated, and thereafter, the compensation model is generated, and the delay compensation unit 82 can be configured using the generated delay model. Note that, although in the above description, machine learning was used to generate the delay model and the compensation model, the present invention is not limited to machine learning, and a mathematical model that describes the phenomena within the condition generator 4 may also be used.

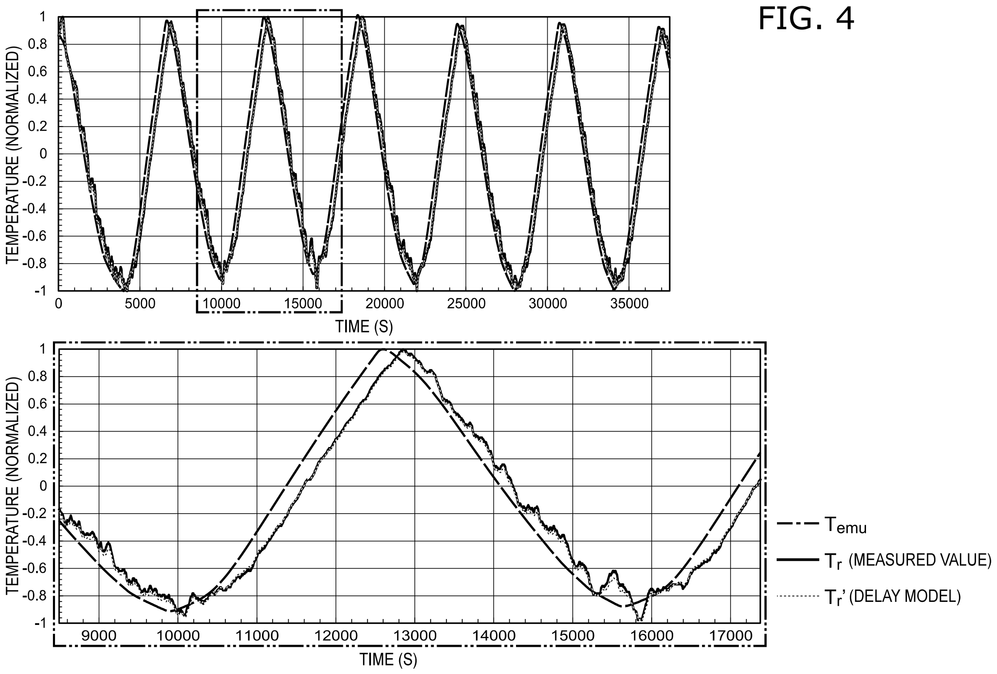

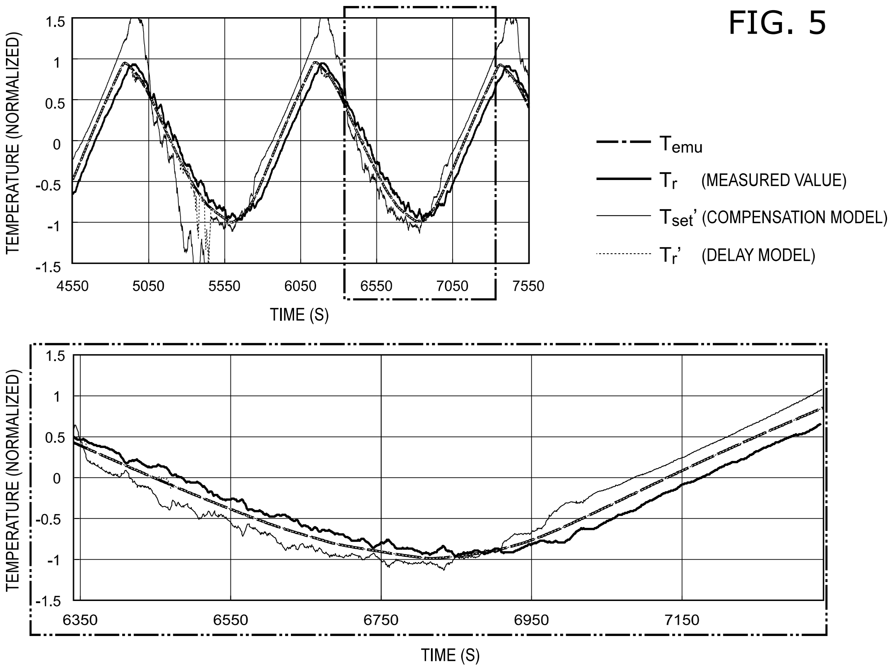

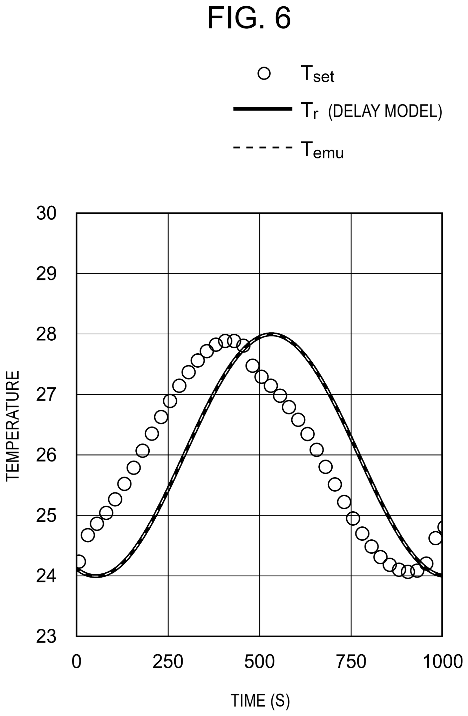

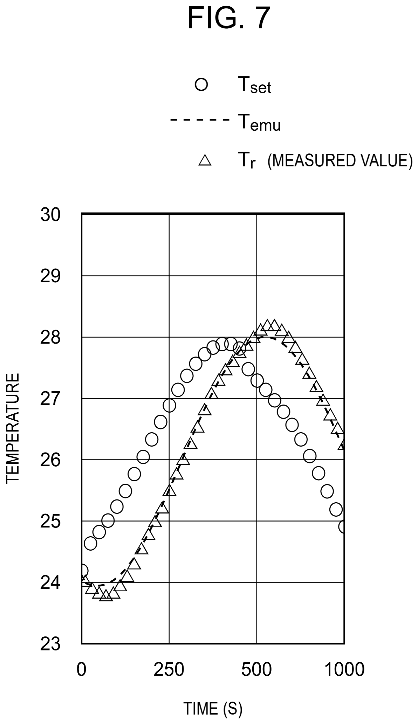

Hereinafter, two specific examples of simulations for the case of configuring the delay compensation unit 82 by machine learning will be described. The first example is illustrated in FIGS. 4 and 5, and involves machine learning using past measurement data, and the performance of the compensation model is verified by simulation. The second example is illustrated in FIGS. 6 and 7 and involves machine learning using explicitly the error of the indoor state of the test room 1 from the air state of the virtual room as a parameter, and the performance of the compensation model is verified by actual measurement. In common to the both examples, the simulations are performed only for temperature but not for humidity.

In the first example, past measurement data was used to generate a delay model indicating the delay of the room temperature Tᵣ with respect to the virtual room temperature Tₑₘᵤ, which is the setting of the condition generator 4. Thereafter, the set temperature Tₛₑₜ of the condition generator 4 is changed to determine such a set temperature Tₛₑₜ' that the difference (Tᵣ^{'} - Tₑₘᵤ) between the room temperature Tr^{'} resulting from the changed set temperature Tₛₑₜ^{'} and the virtual room temperature Tₑₘᵤ of the emulation result becomes minimal (smaller, or a predetermined value or less). As a result, a delay model is generated in which the virtual room temperature Tₑₘᵤ is an input and the set temperature Tₛₑₜ^{'} is an output.

FIG. 4 is a graph showing the validity of a delay model derived from the machine learning in the first example. In this figure, the vertical axis indicates temperature normalized to a number between -1 and +1, and the horizontal axis indicates time in seconds. Furthermore, the dashed-dotted line indicates a virtual room temperature Tₑₘᵤ determined by the emulation unit 81 while the solid line indicates a room temperature Tᵣ within the test room 1 measured by the thermometer 21. The fine dotted line indicates a room temperature Tᵣ' determined by compensating for the delay with respect to the virtual room temperature Tₑₘᵤ using the delay model. Note that in this figure, the portion surrounded by the dashed-double-dotted line rectangle in the upper part is enlarged and shown in the lower part.

As illustrated, the room temperature Tᵣ^{'} indicated by the fine dotted line substantially matches the room temperature Tᵣ inside the test room 1 that has been measured. Since the both values match each other for most of the time, or the difference therebetween is less than or equal to a predetermined value, it can be understood that the delay model has been correctly configured.

FIG. 5 is a graph illustrating the performance of a compensation model generated through machine learning. In this figure, the portion surrounded by the dashed-double-dotted line rectangle in the upper part is enlarged and shown in the lower part.

Similarly to FIG. 4, this figure indicates the virtual room temperature Tₑₘᵤ by a dashed-dotted line. For comparison with FIG. 4, the room temperature Tᵣ is indicated by a solid line. Furthermore, the thin solid line indicates the setting (set temperature Tₛₑₜ^{'}) for the condition generator 4, for which the delay has been compensated with respect to the virtual room temperature Tₑₘᵤ using the compensation model. The thin dotted line indicates the temperature of the test room 1 (room temperature Tᵣ^{'}) generated by simulation using the delay model with this set temperature Tₛₑₜ^{'} as an input. Note that there are many overlapping portions between the dashed-dotted line (virtual room temperature Tₑₘᵤ) and the fine dotted line (room temperature Tᵣ^{'}), and among the small "dots" constituting the fine dotted line, the portions overlapping with the dashed-dotted line are indicated in white.

As illustrated in this figure, the set temperature Tₛₑₜ^{'} is generated with respect to the virtual room temperature Tₑₘᵤ using the compensation model. Then, the result, for the case of using this set temperature Tₛₑₜ^{'} for controlling the condition generator 4 can be derived as a simulation result from the delay model, and the room temperature Tᵣ^{'} as indicated by the thin dotted line is determined.

Since the virtual room temperature Tₑₘᵤ (indicated by the dashed-dotted line) matches the room temperature Tᵣ' (indicated by the fine dotted line) determined using the delay model over much of the time, it can be understood that a compensation model capable of correctly compensating for the delay has been configured. Then, the delay compensation unit 82 configured using this compensation model can suppress the delay of the room temperature Tᵣ with respect to the virtual room temperature Tₑₘᵤ and thus can improve the accuracy of the performance measurement of the air conditioner 2 using the virtual room.

In the second example, a delay model is generated by machine learning using explicitly the error of the room state of the test room 1 from the air state of the virtual room as a parameter. That is, the compensation model generated based on this delay model can directly compensate for not only the delay but also the error.

FIG. 6 is a graph illustrating the performance of the compensation model generated via machine learning in the second example. This figure, compared to the examples of FIGS. 4 and 5, indicates the temperature on the vertical axis without being normalized. The circles indicate the set values (set temperature Tₛₑₜ) for the condition generator 4 generated using the compensation model. The solid line indicates the room temperature Tᵣ determined using the delay model based on these set values (set temperature Tₛₑₜ). The dashed line indicates the virtual room temperature Tₑₘᵤ.

As illustrated, the room temperature Tᵣ indicated by the solid line substantially matches the virtual room temperature Tₑₘᵤ indicated by the dashed line. Since the both values match each other for much of the time, or the difference therebetween is less than a predetermined value, it can be understood that the delay model has been correctly configured.

FIG. 7 is a graph illustrating the measured temperature of the test room 1 in a case where the delay compensation unit 82 generated using the compensation model generated via machine learning was actually used. The circles indicate the set values (set temperature Tₛₑₜ) for the condition generator 4 generated using the compensation model. The dashed line indicates the virtual room temperature Tₑₘᵤ. The triangles indicate the room temperature Tᵣ measured inside the test room 1, which was controlled based on these set values (set temperature Tₛₑₜ).

In the second example as described above, the measured room temperature Tᵣ indicated by the triangles substantially matches the virtual room temperature Tₑₘᵤ indicated by the dashed line. Since the both values match each other for much of the time, or the difference therebetween is less than a predetermined value, it can be understood that the delay has been compensated by the delay compensation unit 82.

According to the method for measuring performances of the air conditioner 2 of the first embodiment, a delay compensation unit 82 compensates for a delay in the change (response) of the air state of the test room 1 (room temperature Tᵣ, room humidity xᵣ) relative to the set values (virtual room temperature Tₑₘᵤ, virtual room humidity xₑₘᵤ) input to the condition generator 4, with respect to the air state of the virtual room (virtual room temperature Tₑₘᵤ, virtual room humidity xₑₘᵤ) determined by the emulation unit 81. Such delay compensation can reduce the difference between the air state of the test room 1 (room temperature Tᵣ, room humidity xᵣ) and the air state of the virtual room (virtual room temperature Tₑₘᵤ, virtual room humidity xₑₘᵤ) and thus can improve the accuracy of the performance measurement of the air conditioner 2.

According to the method for measuring performances of the air conditioner 2 of the first embodiment, the delay compensation unit 82 is operated by feedforward control using the air state of the air blown out from the air conditioner 2 (blowing temperature Tₛ, blowing humidity xₛ, blowing airflow rate Gₛ). Such feedforward control can improve the accuracy of the performance measurement of the air conditioner 2.

According to the method for measuring the performances of the air conditioner 2 of the first embodiment, the air state of the virtual room reproduced by the condition generator 4 includes a virtual room temperature Tₑₘᵤ and a virtual room humidity xₑₘᵤ. Thus, reproduction of both the temperature and humidity of the virtual room in the test room 1 enhances the reproducibility of the measurement conditions for the air conditioner 2 and can improve the accuracy of the performance measurement for the air conditioner 2.

According to the method for measuring the performances of the air conditioner 2 of the first embodiment, a delay model is generated that indicates the delay of the change (response) of the air state (room temperature Tᵣ, room humidity xᵣ) of the test room 1 relative to the state (virtual room temperature Tₑₘᵤ, virtual room humidity xₑₘᵤ) of the virtual room, which becomes the set values for the condition generator 4. Then, by changing the state so that the delay becomes smaller, a setting for the condition generator 4 after the change (virtual room temperature Tₑₘᵤ^{'}, virtual room humidity xₑₘᵤ^{'}) is determined, and a compensation model used for compensating for the delay is generated based on the change. After the generation of the delay model, the compensation model is generated using the generated delay model thereby simplifying the method for configuring the models.

According to the method for measuring the performance of the air conditioner 2 of the first embodiment, compensation for the delay further compensates for an error in the air state of the test room 1 relative to the air state of the virtual room in a case where the delay has been compensated. Thus, by compensating not only for the delay but also for the error, the accuracy of the performance measurement of the air conditioner 2 can be improved.

### (Second Embodiment)

In the first embodiment, the delay compensation unit 82 illustrated in Figure 2 compensates for the delay with respect to the air state of the virtual room (virtual room temperature Tₑₘᵤ, virtual room humidity xₑₘᵤ) that becomes a set value for the condition generator 4 based on the air state in the measuring chamber 3 (blowing temperature Tₛ, blowing humidity xₛ, blowing air volume Gₛ) using a delay model. In the second embodiment, an example of further using other parameters to improve the accuracy of delay compensation will be described.

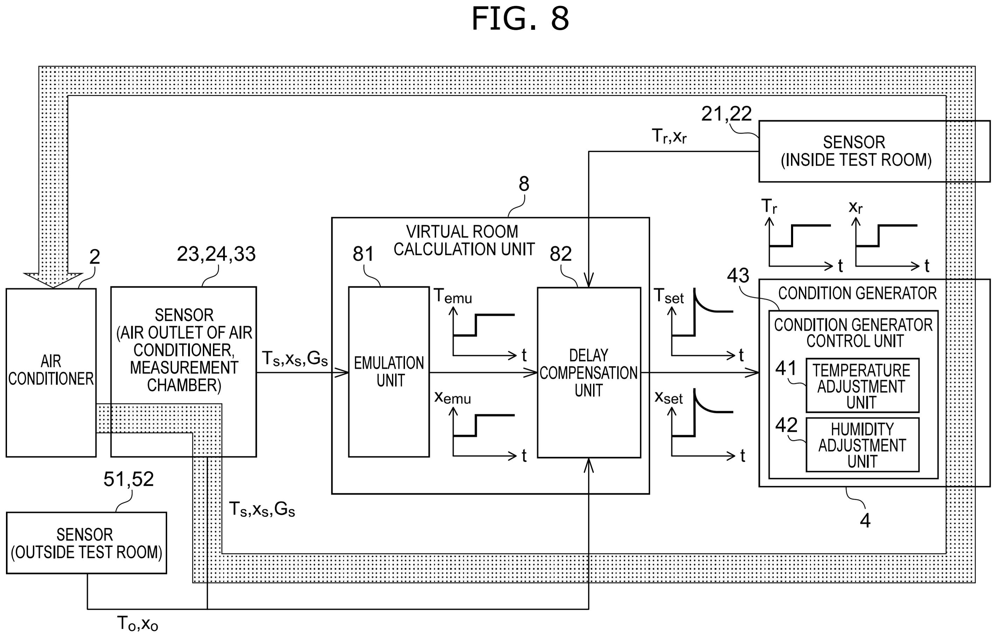

FIG. 8 is an explanatory diagram of the operation of the virtual room calculation unit 8 of the second embodiment. Referring to this figure, input to the delay compensation unit 82 of the virtual room calculation unit 8 are: in addition to the air state (blowing temperature Tₛ, blowing humidity xₛ, blowing air volume Gₛ) of the air blown out from the air conditioner 2, the air state (room temperature Tᵣ, room humidity xᵣ) inside the test room 1 acquired by sensors (thermometer 21, wet-bulb thermometer 22) inside the test room 1, and the air state (outdoor temperature Tₒ, outdoor humidity xₒ) outside the test room 1 acquired by sensors (thermometer 51, wet-bulb thermometer 52) outside the test room 1. The delay compensation unit 82 compensates for the delay with respect to the state (virtual room temperature Tₑₘᵤ, virtual room humidity xₑₘᵤ) of the virtual room based on these inputs and uses what has been compensated as the set values (set temperature Tₛₑₜ, set humidity xₛₑₜ) of the condition generator 4.

The delay in the response of the air state within the test room 1 relative to the set values input to the condition generator 4 may be considered to be caused by heat conduction/heat radiation from outside the test room 1 via the heat insulating material 10, in addition to delays caused by the control of the condition generator 4, the heat capacity of the air within the test room 1, and the like. In particular, since the outdoor unit 5 is increased in temperature, a feedforward using the outdoor temperature Tₒ and outdoor humidity xₒ measured by the thermometer 51 and wet-bulb thermometer 52 provided near the outdoor unit 5 is used to compensate for the delay due to the heat conduction/heat radiation from outside the test room 1.

Furthermore, the delay compensation unit 82 can directly determine the delay/advance of the response of the air state (room temperature Tᵣ, room humidity xᵣ) inside the test room 1 with respect to the set values (set temperature Tₛₑₜ, set humidity xₛₑₜ) of the condition generator 4 by accepting the air state (room temperature Tᵣ, room humidity xᵣ) inside the test room 1.

Therefore, when the air state inside the test room 1 (room temperature Tᵣ, room humidity xᵣ) is delayed with respect to the set values (set temperature Tₛₑₜ, set humidity xₛₑₜ) of the condition generator 4, the level of compensation is further increased, and when the air state inside the test room 1 (room temperature Tᵣ, room humidity xᵣ) is advanced with respect to the set values (set temperature Tₛₑₜ, set humidity xₛₑₜ) of the condition generator 4, the level of compensation is decreased. In this manner, the delay compensation unit 82 can optimally compensate for the delay by feedback control using the air state inside the test room 1 (room temperature Tᵣ, room humidity xᵣ).

In this embodiment, the delay compensation unit 82 compensates for the delay using the air state inside the test room 1 (room temperature Tᵣ, room humidity xᵣ) and the air state outside the test room 1 (outdoor temperature Tₒ, outdoor humidity xₒ) in addition to the air state inside the measuring chamber 3 (blowing temperature Tₛ, blowing humidity xₛ, blowing air volume Gₛ). Even in such a case, machine learning can generate the delay model and the compensation model by increasing the number of the parameters handled during model generation.

According to the method for measuring the performance of the conditioner 2 of the second embodiment, the delay compensating unit 82 further compensates for the delay using feedback control based on the air state inside the test room 1 (room temperature Tᵣ, room humidity xᵣ). Such feedback control can appropriately adjust the level of delay compensation, thereby further improving the accuracy of performance measurement for the air conditioner 2.

According to the method for measuring the performance of the conditioner 2 of the second embodiment, the delay compensating unit 82 further compensates for the delay using feedforward control based on the air state outside the test room 1 (outdoor temperature Tₒ, outdoor humidity xₒ). Such feedforward control using the state of the air blown out from the air conditioner 2 (blowing air temperature Tₛ, blowing air humidity xₛ, and blowing air volume Gₛ) can appropriately adjust the level of delay compensation, thereby further improving the accuracy of performance measurement for the air conditioner 2.

The present invention allows for various embodiments and modifications without departing from the broad spirit and scope of the present invention. Further, the embodiments described above are for explaining the present invention, and do not limit the scope of the present invention. That is, the scope of the present invention is indicated not by the embodiments but by the scope of claims. Various modifications made within the scope of claims and the scope of the significance of the invention equivalent thereto are regarded as being within the scope of the present invention.

### Reference Signs List

- 1: Test room
- 2: Air conditioner
- 3: Measuring chamber
- 4: Condition generator
- 5, 6: Outdoor unit
- 7: Air conditioning performance measurement unit
- 8: Virtual room calculation unit
- 9: Performance measurement apparatus
- 21, 23, 31, 51: Thermometer
- 24: Hygrometer
- 22, 32, 52: Wet-bulb thermometer
- 25, 53: Power meter
- 33: Air flow meter
- 81: Emulation unit
- 82: Delay compensation unit

## Claims

1. A method for measuring the performance of an air conditioner, the air conditioner being dynamically operated in a test room equipped with a condition generator that generates measurement conditions, the method comprising:
emulating the air state of a virtual room, the virtual room being a virtual reproduction of an actual room, using the air state of the air blown out from the air conditioner to determine future changes in the air state of the virtual room;
compensating for a delay in the air state of the test room relative to a setting of the condition generator in the case of using the future change in the air state of the virtual room for the setting of the condition generator;
controlling the condition generator using, as the setting, the future change in the air state of the virtual room for which the delay has been compensated; and
measuring the performance of the air conditioner in the test room under the measurement conditions generated by the condition generator.

2. The method for measuring the performance of an air conditioner according to claim 1, wherein the delay is compensated by feedforward control using the air state of the air blown out from the air conditioner.

3. The method for measuring the performance of an air conditioner according to claim 2, wherein the delay is further compensated by feedback control using the air state inside the test room.

4. The method for measuring the performance of an air conditioner according to claim 2 or 3, wherein the delay is further compensated by feedforward control using the air state outside the test room.

5. The method for measuring the performance of an air conditioner according to claim 1, wherein the air state comprises at least either one of the temperature or humidity of the air.

6. The method for measuring the performance of an air conditioner according to claim 1, wherein in the compensation for the delay, an error of the air state of the test room relative to the air state of the virtual room for which the delay has been compensated is further compensated for.

7. The method for measuring the performance of an air conditioner according to claim 1, comprising:
generating a delay model that indicates the delay of the air state of the test room relative to the setting of the condition generator; and
changing the setting for the condition generator so that the delay becomes smaller, and generating a compensation model used for the compensation of the delay based on said change.

8. An apparatus for measuring the performance of an air conditioner, the air conditioner being dynamically operated in a test room equipped with a condition generator that generates measurement conditions, the apparatus comprising:
an emulation unit configured to emulate the air state of a virtual room, the virtual room being a virtual reproduction of an actual room, using the air state of the air blown out from the air conditioner to determine future changes in the air state of the virtual room;
a delay compensation unit configured to compensate for a delay in the air state of the test room relative to a setting of the condition generator in the case of using the future change in the air state of the virtual room for the setting of the condition generator and to control the condition generator using, as the setting, the future change in the air state of the virtual room for which the delay has been compensated; and
an air conditioning performance measurement unit configured to measure the performance of the air conditioner in the test room under the measurement conditions generated by the condition generator.

9. A method for controlling a condition generator, the condition generator being installed in a test room and generating conditions for performance measurement of an air conditioner dynamically operated, the method comprising:
emulating the air state of a virtual room, the virtual room being a virtual reproduction of an actual room, using the air state of the air blown out from the air conditioner to determine future changes in the air state of the virtual room;
compensating for a delay in the air state of the test room relative to a setting of the condition generator in the case of using the future change in the air state of the virtual room for the setting of the condition generator; and
controlling the condition generator using, as the setting, the future change in the air state of the virtual room for which the delay has been compensated.
